# EUROPEAN PATENT APPLICATION

(11) **EP 0 681 816 A2**
(43) Date of publication of application: **15.11.1995**
(21) Application number: 95201182.3
(22) Date of filing: 08.05.1995
(51) Int. Cl.: A61F 2/36, A61F 2/30

(54) **Femoral part of a hip joint endoprosthesis**

(30) Priority: 07.05.1994 DE 4416278
(71) Applicant: ESKA Implants GmbH, 23556 Lübeck (DE)
(72) Inventor: Henssge, Ernst-Joachim, D-23562 Lübeck (DE); Thomas, Wolfram, I-00135 Rom (IT); Scholz, Jörg, D-14193 Berlin (DE); Schug, Martin, D-23552 Lübeck (DE)
(74) Representative: Weiss, Christian, Dipl.-Ing.

(57) **Abstract**

A femoral part (1) of a hip joint endoprosthesis has a proximal prosthesis section (2) and a distal prosthesis section (20) tapering toward the distal end (26). The proximal prosthesis section (2) may have an open-cell or open-pore surface structure (12) and the distal section (20) has a media longitudinal groove (28) in order to impart greater elasticity to the shaft on its distal end (26).

## Description

The invention concerns a femoral part of a hip joint endoprosthesis with a proximal prosthesis section and a distal prosthesis section that tapers toward the distal end.

This type of femoral part is known from DE 41 25 152 C2, which has an open-pore or open-cell surface structure on the shaft of the proximal prosthesis section to improve the implant/bone interface, whereas the distal prosthesis section is smooth.

A two-part femoral part of a hip joint endoprosthesis is known from EP 0 307 646 B1. Division into a proximal prosthesis section and a distal prosthesis section that can be connected together by means of a tapered plug-in connector has the purpose in this known design to permit flexible adjustment of the shaft to the needs of different patients with respect to length and diameter without requiring costly stock-keeping on the part of the hospital. Since both the proximal prosthesis section and the distal prosthesis section of this known endoprosthesis are designed as a full cross section, the growth conditions, long-term stability and adjustment to the elasticity conditions of the natural femur are not satisfactory in all cases.

The object of the invention is therefore to modify a femoral part of the type mentioned in the introduction so that the ingrowth behaviour and long-term stability of the implant are improved.

This object is achieved according to the invention in the femoral part of the type mentioned in the introduction in that the proximal prosthesis section has a surface structure adapted to allow bony ingrowth at least in sections on the proximal section and that the distal prosthesis section has a medial longitudinal groove.

The advantages of the invention lie especially in the fact that the distal prosthesis section has a medial longitudinal groove that lessens the shaft cross section and imparts greater elasticity to the distal prosthesis section, which is better matched to the elasticity of the natural femur. In this fashion a femoral part is achieved whose proximal prosthesis section is rigidly held by spongy tissue growing into the open surface structure, whereas the rigidity of the distal prosthesis section is reduced in desirable fashion by appropriate dimensioning of the medial longitudinal groove. Thus undesired proximal stress shielding phenomena are reduced or avoided according to the invention because the distal stress concentration is reduced by the increased elasticity.

It is particularly preferred that the longitudinal groove extends in the mediallateral direction nearly to the centre of the shaft cross section or beyond the centre of the shaft cross section; the longitudinal groove occupies a peripheral section of at least 1/10 of the entire periphery in the peripheral direction on the surface, advantageously more than 1/5 of the total periphery. In this embodiment according to the invention the shaft cross section of the distal prosthesis section can be designed to have a crescent-shape, in which the concave opening of the crescent faces the medial direction and the convex back of the crescent faces laterally. Depending on the shape and depth of the medial longitudinal groove, the original rigidity of the shaft cross section can be reduced in desirable fashion and a desired elasticity of the distal end of the shaft achieved.

The longitudinal groove preferably extends over the entire length of the distal part of the shaft, but alternatively the longitudinal groove can also be formed only over a stipulated length section on the distal end of the distal prosthesis section. The shaft cross section then gradually reassumes its full cross section at a stipulated distance in front of the proximal prosthesis section, which continues into the proximal prosthesis section step-free.

According to an advantageous embodiment of the invention the proximal prosthesis section and the distal prosthesis section are each designed as separate parts and both parts are preferably joinable by a tapered plug-in connection. Because of the two-part nature of the prosthesis it is possible according to the invention to combine femoral parts in different axial length from a few different proximal prosthesis parts and a few different distal prosthesis parts with overall reduced stock-keeping so that a number of different prostheses customised to the patient can be assembled with overall reduced stock-keeping.

Advantageously it is also further possible to divide the proximal prosthesis section into two separate segments, a collar/neck segment and a proximal shaft segment in order to be able to adapt to additional prosthesis parameters, such as the CCD angle, the individual circumstances of the patient - with overall reduced stock-keeping.

According to a preferred embodiment of the invention the boundary surface between the proximal prosthesis section and the distal prosthesis section runs perpendicular to the shaft axis; alternatively, however, the boundary surface can also assume a curved form so that, for example, the open surface structure of the proximal prosthesis section extends farther in the medial region toward the distal shaft and then in the lateral region in order to improve growth of the spongy tissue medially and to permit more possibility of movement relative to the bone laterally around the shaft. This type of embodiment of the boundary surface results in a situation in which stronger relative movement is permitted between the shaft and bone in the lateral region with sufficient ingrowth of the bone so that the stress transfer to the proximal section of the femur is reduced.

Alternatively, it is also possible to impart an open surface structure to the distal prosthesis section, at least in sections, if, for example, in a boundary surface that runs perpendicular to the shaft axis the ingrowth zone is to be increased medially to the distal end.

Advantageous modifications of the invention are characterized by the features of the subclaims.

A practical example of the invention will be explained further below with reference to the drawings. In the drawings:
- Figure 1: shows a side view of a femoral part of a hip joint endoprosthesis;
- Figure 2: shows a cross section through the femoral part along line A-A;
- Figure 3: shows a cross section through the femoral part of Figure 1 along line B-B;
- Figure 4: shows a cross section through the femoral part of Figure 1 along line C-C;
- Figure 5: shows a side view of an alternative embodiment of the femoral part and
- Figure 6: shows a cross section through an additional alternative embodiment of the femoral part.

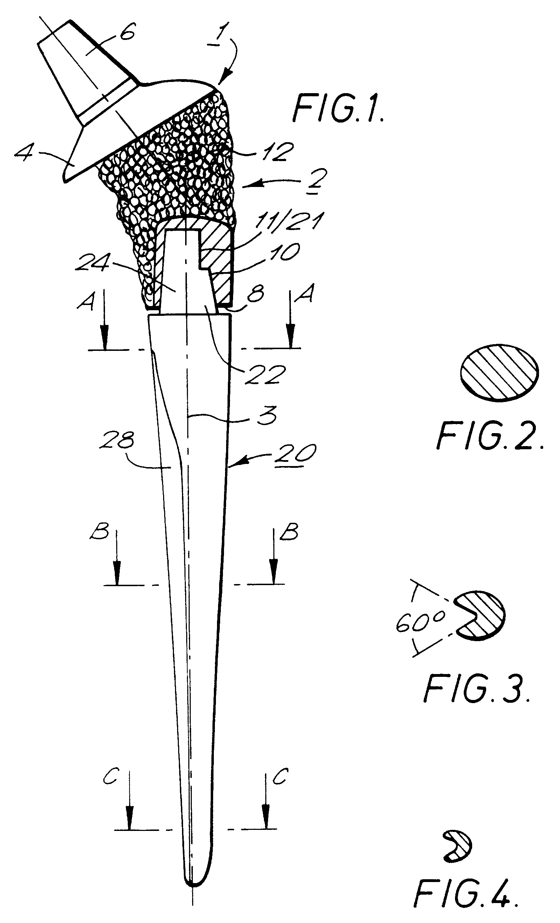

Figures 1 to 4 show a femoral part **1** of a hip joint endoprosthesis that has a proximal prosthesis section **2** and a distal prosthesis section **20**, which represent separate parts in the variant according to Figures 1 to 4 and can be joined by means of a tapered plug-in connector **24**, **10**. The proximal prosthesis section **2** has a collar **4** that runs at a stipulated angle to the shaft axis **3** and carries a tapered pin **6** on which a spherical prosthetic joint head (not shown) can be mounted. Alternatively, the prosthetic joint head can also be formed in one piece with the proximal prosthesis section **2**.

According to Figure 1 the proximal prosthesis section **2** has a boundary surface **8** to the distal prosthesis section **20** that runs perpendicular to shaft axis **3** and in which a conical recess **10** is made concentric to shaft axis **3**. A shoulder 11 is provided in conical recess **10** on one point of the conical periphery, which meshes with a corresponding recess **21** of tapered pin **24** during connection of the tapered plug-in connector and in this fashion guarantees rotational fixation. The proximal prosthesis section **2** is provided with an open surface structure, preferably of the open-celled or open-pored type although any open surface allowing the ingrowth of bone would be suitable for use in the present invention **12**, beneath collar **4** in which the adjoining spongy tissue can grow following implantation. The open-cell or open-pore surface structure **12** extends on the periphery of proximal shaft section **2** from collar **4** to boundary surface **8** in the embodiment shown.

The distal prosthesis section **20** also has a boundary surface **22** on which a tapered pin **24** is formed concentrically to shaft axis **3**. The tapered pin **24** meshes in shape-mated and self-inhibiting fashion with the conical recess **10** of the proximal prosthesis section **2** and in turn has a recess **21** that meshes with the corresponding shoulder **11** in the conical recess **10** of proximal prosthesis section **2** and forms a rotational fixation.

The distal prosthesis section **20** has a media longitudinal groove **28** that starts at a limited distance from boundary surface **22** and extends in the media-lateral direction to the centre of the shaft cross section through which the shaft axis **3** runs. The longitudinal groove **28** occupies at least 1/10 of the total periphery of the distal stem with more than 1/5 of the total periphery in the embodiment shown. The longitudinal groove **28** is designed so that the distal prosthesis section **20** has a crescent-shaped cross section that diminishes in terms of surface toward the distal end. The longitudinal groove **28** is made so that only rounded longitudinal edges are formed in the shaft cross section.

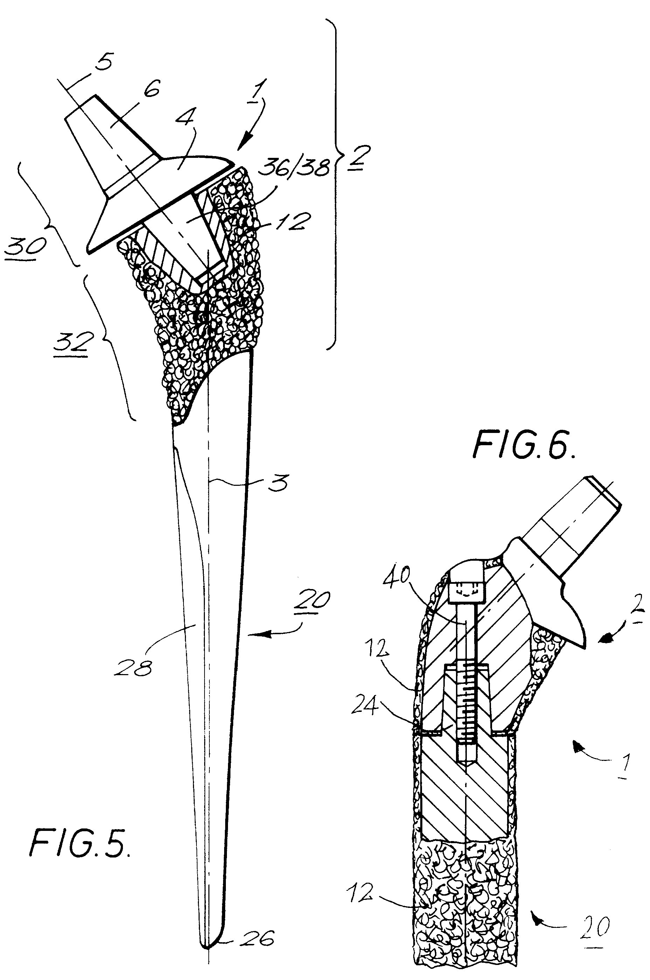

Figure 5 shows a side view of another embodiment of a femoral part **1** that essentially corresponds to the embodiment depicted in Figures 1 to 4 except that in the embodiment according to Figures 1 to 4 the proximal prosthesis section **2** and the distal prosthesis section **20** according to Figure 5 merge in one piece and the open-cell or open-pore surface structure **12** arranged beneath collar **4** on the shaft extends farther downward medially toward the distal prosthesis end **26** than in the lateral region so that a curved boundary line is formed between the proximal prosthesis section **2** and the distal prosthesis section **20**.

According to Figure 5 the proximal prosthesis section **2** is divided into a separate collar/neck segment **30** and a proximal shaft segment **32** connected to it. A tapered plug-in connector **36**, **38** is provided between collar/neck segment **30** and the proximal shaft segment **32**, which consists of a cone **36** formed on the collar/neck segment **30** and a corresponding conical hole **38** in shaft segment **32**. In the embodiments shown the cone **36** and the conical hole **38** run coaxially to the neck axis **5** running through the conical pin **6**. Alternatively, the axis of the tapered plug-in connection **36**, **38** can have a parallel offset or an angular offset relative to neck axis **5** in order to permit better adjustment of the shape of the prosthesis to the circumstances of the individual patients.

Figure 6 is a cross section of a further embodiment of the invention where the proximal and medial parts of the hip joint endoprosthesis are secured together by means of a locking bolt **40** which passes through the proximal section **2** and into the distal section **20**. Both sections are provided with an open-celled or open-pored surface structure **12**.

## Claims

1. Femoral part of a hip joint endoprosthesis having a proximal prosthesis section and a distal prosthesis section tapering toward its distal end, characterized in that the distal prosthesis section (20) has a media longitudinal groove (28).

2. Femoral part according to Claim 1, characterized in that the longitudinal groove (28) extends laterally nearly to the centre of the shaft cross section.

3. Femoral part according to Claim 1, characterized in that the longitudinal groove (28) extends laterally beyond the centre of the shaft cross section.

4. Femoral part according to one of the preceding Claims, characterized in that the longitudinal groove (28) occupies a peripheral section on the surface of at least 1/10 of the total periphery.

5. Femoral part according to one of the preceding Claims, characterized in that the longitudinal groove (28) occupies a peripheral section on the surface of the distal prosthesis section (20) that amounts to at least 1/5 of the total periphery.

6. Femoral part according to one of the preceding Claims, characterized in that the distal prosthesis section (20) has a crescent-shaped shaft cross section toward the distal end (26).

7. Femoral part according to one of the preceding Claims, characterized in that the longitudinal groove (28) extends over the entire length of the distal prosthesis section (20).

8. Femoral part according to one of the preceding claims characterised in that the proximal prosthesis section (2) has an open surface structure (12) at least partially covering the proximal prosthesis.

9. Femoral part according to one of the preceding Claims, characterized in that the distal prosthesis section (20) has a crescent-shaped cross section over the entire length of longitudinal groove (28).

10. Femoral part according to one of the preceding Claims, characterized in that the boundary surface (8, 22) runs perpendicular to shaft axis (3) between the proximal prosthesis section (2) and the distal prosthesis section (20).

11. Femoral part according to one of the Claims 1 to 10, characterized in that the open surface structure is an open-pore or open-cell surface structure (12) which extends to boundary surface (8) on the entire periphery of the bottom of collar (4).

12. Femoral part according to one of the preceding Claims, characterized in that the distal shaft part (20) has as least one section with an open-pore or open-cell surface structure.

13. Femoral part according to Claim 12, characterized in that the section of the distal shaft part (20) provided with the open-pore or open-cell surface structure (12) abuts the proximal prosthesis section (2).

14. Femoral part according to one of the preceding Claims, characterized in that the proximal prosthesis section (2) and the distal prosthesis section (20) are separate parts adapted to be joined together.

15. Femoral part according to Claim 14, characterized in that the proximal prosthesis section (2) and the distal prosthesis section (20) can be joined by means of a tapered plug-in connection (10, 24).

16. Femoral part according to one of the preceding Claims, characterized in that the proximal prosthesis section (2) is divided into a collar/neck segment (30) and a proximal shaft segment (32).

17. Femoral part according to Claim 16, characterized by a tapered plug-in connection between the collar/neck segment (30) and the proximal shaft segment (32).

18. Femoral part according to one of the preceding Claims, characterized in that the cross-sectional area of the longitudinal groove (28) increases toward the distal end.
